# EUROPÄISCHE PATENTANMELDUNG

(11) **EP 3 680 681 A1**
(43) Veröffentlichungstag der Anmeldung: **15.07.2020**
(21) Anmeldenummer: 19151172.4
(22) Anmeldetag: 10.01.2019
(51) Int. Cl.: G01R 33/561, G01R 33/54

(54) **AUSWAHL EINES ABTASTSCHEMAS FÜR EINE MAGNETRESONANZFINGERPRINTING-AUFNAHME UND EINES ENTSPRECHENDEN WÖRTERBUCHS BASIEREND AUF EINER VORABAUFNAHME**

(71) Anmelder: Siemens Healthcare GmbH, 91052 Erlangen (DE)
(72) Erfinder: Kartäusch, Ralf, 91058 Erlangen (DE); Zeller, Mario, 91054 Erlangen (DE)

(57) **Zusammenfassung**

Die Erfindung betrifft ein Verfahren zur Steuerung eines Magnetresonanztomographie-Systems (1) im Rahmen einer MRF-Messung umfassend die Schritte:
- Erstellung oder Bereitstellung einer Dictionary-Gruppe (DG) umfassend mindestens zwei Dictionarys (D1, D2), wobei jedes Dictionary D1, D2 eine Vielzahl von unterschiedlichen Intensitätsverläufen (I) einer Serie von MRT-Aufnahmen (B1, B2, B3, B4) mit einem spezifischen Abtastschema (A) enthält,
- Anfertigen einer Vorabaufnahme (V) von MRT-Messungen,
- Ermittlung und Festlegung eines Abtastschemas (A) basierend auf der Vorabaufnahme (V),
- Auswahl eines Dictionarys (D1, D2) aus der Dictionary Gruppe (DG) basierend auf der Vorabaufnahme (V),
- Durchführung einer MRF-Messung mit dem festgelegten Abtastschema (A) und MRF-Auswertung basierend auf dem ausgewählten Dictionary (D1, D2).

Die Erfindung betrifft darüber hinaus eine entsprechende Vorrichtung, eine entsprechende Steuereinrichtung und ein Magnetresonanztomographie-System, sowie ein Verfahren zur Herstellung eines Magnetresonanztomographie-Systems.

## Beschreibung

Die Erfindung betrifft ein Verfahren und eine Vorrichtung zur Steuerung eines Magnetresonanztomographie-Systems im Rahmen einer MRF-Messung, eine entsprechende Steuereinrichtung und ein Magnetresonanztomographie-System ("MRT-System"), sowie ein Verfahren zur Herstellung eines Magnetresonanztomographie-Systems.

Magnetic Resonance Fingerprinting (MRF) ist seit einiger Zeit eine vielversprechende neue Technologie für quantitative Bildgebung. Bei Durchführung eines MRF-Verfahrens wird ein Intensitätsverlauf über die Zeit gemessen. Dabei wird während einer Messung mittels Hochfrequenzpulsen mit variierenden Flipwinkeln und/oder Abständen hinsichtlich der Echozeit (TE) oder Repetitionszeit (TR) eine möglichst hohe Dynamik des Intensitätsverlaufs unterschiedlicher Aufnahmen erzeugt. Die Reihenfolge, welche Aufnahmen mit welchen Parameterwerten erfolgen, wird als "Abtastschema" bezeichnet. Bezogen auf die Ansteuerung eines MRT-Systems kann das Abtastschema als die Anordnung von Signalen in einer Pulssequenz oder die Reihenfolge verschiedener Pulssequenzen angesehen werden.

Hierbei werden einzelne Pixel von Bildern betrachtet. Nimmt man an, es wird von demselben Bereich eines Patienten mit unterschiedlichen Parametern mehrfach ein Bild bestehend aus Bildpunkten aufgenommen, so würden nun jeweils diejenigen Bildpunkte, die einer gemeinsamen Raumkoordinate des Bereichs entsprechen, zu einer Gruppe zusammengefasst und deren Intensitätsverlauf über die verschiedenen Aufnahmen hinweg betrachtet. Im Folgenden wird aus Gründen der besseren Übersicht und da oftmals Stapel von zweidimensionalen Bildern vorliegen, diese Gruppen aus Bildpunkten als "Pixel" bezeichnet. Dieser Ausdruck "Pixel" bzw. "pixelweise" ist also nicht nur auf einen Bildpunkt eines einzigen Bildes bezogen, sondern für die Gruppe aller Bildpunkte einer Bilderserie an einer (für jede Gruppe stets gleichen) Raumkoordinate bzw. Bildkoordinate. Geben alle Bilder einer betreffenden Bilderserie stets denselben Bereich wieder, entspricht dieselbe Bildkoordinate derselben Raumkoordinate. Im Grunde gibt jedes Pixel den Zustand eines Punktes in dem Patienten an, der mit unterschiedlichen Parametern aufgenommen worden ist. Ein pixelweiser Intensitätsverlauf kann also in der Regel als Intensitätsverlauf an einem speziellen Pixel über die Bilderserie angesehen werden. Der Intensitätsverlauf wird nun pixelweise (mit derselben Raumkoordinate) für alle Aufnahmen betrachtet.

Nach einer Fourier-Transformation, in der Regel ausgeführt als Non Uniform FFT (da oftmals ein stark unterabgetastetes Bild mittels Spiral-Trajektorie aufgenommen wird) wird der Intensitätsverlauf pixelweise mit Einträgen eines zuvor simulierten "Wörterbuchs" verglichen. Es hat sich in der Bildgebung auch im Deutschen ergeben, dass der englische Begriff "Dictionary" verwendet wird. Das Dictionary ist eine Sammlung von Simulationen von Intensitätsverläufen des Gewebes mit dem Abtastschema der Sequenz. Dabei wird eine Vielzahl von Werten gewebespezifischer Parameter, beispielsweise T1- und T2-Relaxationszeiten sowie Protonendichten für das Gewebe simuliert. Die Gesamtheit dieser Simulationen für verschiedene T1 und T2 Werte stellt dann die Einträge des Dictionary dar. Es kann zusammenfassend gesagt werden, dass ein Eintrag des Dictionary einem Intensitätsverlauf in Abhängigkeit der betrachteten, z.B. gewebespezifischen, Parameter gemäß dem vorgegebenen Abtastschema entspricht.

Es erfolgt nun für eine Bilderserie für jedes Pixel ein Vergleich des Intensitätsverlaufs mit Einträgen des Dictionary, welche zumeist verschiedenen T1 und T2 Werten entsprechen. Der Vergleich der gemessenen Intensitätsverläufe mit den Intensitätsverläufen des Dictionary sucht den ähnlichsten Intensitätsverlauf für jedes Pixel. Es wird also für jeden pixelweisen Intensitätsverlauf derjenige Eintrag des Dictionary verwendet, bei dem der (simulierte) Intensitätsverlauf am besten mit dem realen Intensitätsverlauf übereinstimmt. Für diesen Intensitätsverlauf werden dann die gewebespezifischen Parameter, also z.B. T1- und T2-Werte Pixel für Pixel in einer Karte abgespeichert.

Dies wird dann mit weiteren Pixeln wiederholt. Führt man dies für alle Pixel durch, erhält man eine T1 und T2 Karte.

Das bisherige Vorgehen ist der Vergleich mit einem einzigen Dictionary und ein fixes Abtast-/Anregungsschema welches verwendet wird, weil es in initialen Experimenten passable Ergebnisse lieferte.

Voraussetzung für eine gute Differenzierung der verschiedenen Magnetresonanzparameter ("MR-Parameter") ist ein möglichst spezifisches Abtastschema des Signalverlaufs. Dafür wird z.B. versucht TR und TE zu variieren. Die Genauigkeit des Verfahrens hängt dabei explizit vom Abtastschema ab. Ein gravierendes Problem dabei ist ein optimales Schema zu finden, um möglichst schnell die gewebespezifischen MR Kenngrößen zu bestimmen (z.B. T2, T1 und Diffusion).

Es ist eine Aufgabe der vorliegenden Erfindung, ein alternatives, komfortableres Verfahren und eine entsprechende Vorrichtung sowie eine Steuereinrichtung zur Steuerung eines Magnetresonanztomographie-Systems im Rahmen einer MRF-Messung und ein entsprechendes Magnetresonanztomographie-System anzugeben, mit dem die oben beschriebenen Nachteile vermieden werden.

Diese Aufgabe wird durch ein Verfahren gemäß Patentanspruch 1, eine Vorrichtung gemäß Patentanspruch 10, eine Steuereinrichtung gemäß Patentanspruch 11, ein Magnetresonanztomographie-System gemäß Patentanspruch 12, sowie ein Verfahren zur Herstellung eines Magnetresonanztomographie-Systems gemäß Patentanspruch 13 gelöst.

Das erfindungsgemäße Verfahren sowie die erfindungsgemäße Vorrichtung dienen zur Steuerung eines Magnetresonanztomographie-Systems im Rahmen einer MRF-Messung. Sie können insbesondere in Form von Komponenten vorliegen, die als ergänzende Komponenten in ein Magnetresonanztomographie-System integriert werden können.

Das erfindungsgemäße Verfahren umfasst die folgenden Schritte:
- Erstellung oder Bereitstellung einer Dictionary-Gruppe umfassend mindestens zwei Dictionarys, wobei jedes Dictionary eine Vielzahl von unterschiedlichen Intensitätsverläufen, die einer Serie von MRT-Aufnahmen mit einem spezifischen Abtastschema entsprechen, enthält.

Bevorzugt umfasst die Dictionary-Gruppe mehr als zwei Dictionarys, bevorzugt mehr als 4 Dictionarys, wobei die Dictionarys z.B. speziell für unterschiedliche Körperregionen erstellt wurden. Jedes Dictionary umfasst als Einträge die besagten Intensitätsverläufe, die innerhalb eines Dictionarys alle mit demselben Abtastschema aufgenommen worden sind. Unterschiedlichen Dictionarys kann zwar durchaus dasselbe Abtastschema (aber z.B. andere gesuchte Parameter (T1, T2) oder andere Parameterwerte) zugrunde liegen, bevorzugt ist jedoch, dass mindestens 2 Dictionarys der Dictionary-Gruppe auf unterschiedlichen Abtastschemata basieren.

Ein Abtastschema gibt die Reihenfolge der Aufnahmen bezüglich der Zeit zusammen mit der erfolgten Variation der Aufnahmeparameter (s.o. Flipwinkel, TE, TR, etc.) wieder. Die Variation kann theoretisch (zumindest Abschnittsweise) marginal sein, so dass immer wieder derselbe Parameterwert verwendet wird, jedoch ist für eine optimale Auswertung bevorzugt, dass eine Variation von Parametern vorgenommen wird, so dass eine deutlich messbare Variation in der Intensität eines Pixels über die unterschiedlichen Aufnahmen messbar ist. Es ist von Vorteil, wenn innerhalb eines sinnvollen Bereichs der Parameterwerte eine möglichst große Schwankung der Intensität hervorgerufen wird. Eine hohe Dynamik des Intensitätsverlaufs führt zu einer optimalen Vergleichbarkeit mit Einträgen eines Dictionarys. Es kann in einer nachfolgenden Aufnahme im Vergleich mit der vorangehenden Aufnahme nur ein einziger Parameterwert geändert werden aber auch zwei oder mehr.

Bevorzugt sind in einem Dictionary Informationen zu demjenigen Abtastschema enthalten, auf dem dessen Einträge basieren. Das betreffende Abtastschema kann aber auch anders mit dem Dictionary verknüpft sein, z.B. durch eine Referenz, eine Verlinkung oder eine Codierung. Informationen über das Abtastschema können auch mit einer Adresse des Dictionary in der Dictionary-Gruppe verknüpft sein. Die Hauptsache dabei ist, dass mit der Auswahl eines Dictionary aus der Dictionary-Gruppe auch das spezifische Abtastschema des ausgewählten Dictionary ermittelt werden kann.

Ein Abtastschema ist also spezifisch für jedes Dictionary, wobei jedoch innerhalb eines Dictionarys alle Einträge auf demselben Abtastschema basieren. Die Erfindung nutzt die Tatsache, dass unterschiedliche Abtastschemata unterschiedlich gut für die verschiedenen gesuchten Parameter (z.B. T1 und T2) von unterschiedlichen Körperregionen anwendbar sind.
- Anfertigen einer Vorabaufnahme von MRT-Messungen. Diese MRT-Messungen können einfach so gestaltet sein, dass Aussagen über die Parameterreichweite getroffen werden können, sie können aber auch eine Mehrzahl von MRT-Aufnahmen mit variierenden Aufnahmeparameterwerten umfassen. Für diese Vorabaufnahme kann auch der englische Ausdruck "Prescan" verwendet werden. Die Vorabaufnahme wird vor der eigentlichen MRF Messung durchgeführt, um die Verteilung der gesuchten Parameterwerte (z.B. von T2 und T1 Werten) in der gewünschten Körperregion zu ermitteln.

Die Vorabaufnahme kann beispielsweise eine Messung sein, die möglichst schnell einen Überblick über die Parameterreichweite (z.B. Minimum und Maximum) der gesuchten untersuchungsobjektspezifischen Parameter, z.B. über die aufgenommenen Kontraste (z.B. T1 und T2), gibt. Nicht-quantitative MR-Verfahren können eine Wichtung gemäß einem gewünschten Kontrast, z.B. T1-Wichtung oder T2-Wichtung, aufweisen. Dabei kann der Kontrast auf eine bestimmte Parameterreichweite, einen Wertebereich, des jeweiligen gewebespezifischen Parameters eingestellt sein. Somit können auch aus einer nichtquantitativen Vorabaufnahme Rückschlüsse auf in dem untersuchten Objekt vorhandene Parameterwerte gezogen werden.

Da durch die Vorabaufnahme der Wertebereich bekannt ist, kann nun gezielt ein optimales Dictionary (mit seinem entsprechenden Abtastschema und/oder mit einem passenden Wertebereich der gesuchten Parameter in den in dem Dictionary umfassten Intensitätsverläufen) verwendet werden.
- Ermittlung und Festlegung eines Abtastschemas basierend auf der Vorabaufnahme und
- Auswahl eines Dictionary aus der Dictionary Gruppe basierend auf der Vorabaufnahme.

Die Festlegung eines Abtastschemas kann dabei direkt aus der Vorabaufnahme erfolgen, aber auch nach Auswahl eines Dictionary aus der Dictionary-Gruppe entsprechend dem Abtastschema des ausgewählten Dictionary. Wenn das Abtastschema vor der Auswahl des Dictionary erfolgt, kann theoretisch die MRF-Messung ebenfalls vor Auswahl des Dictionary erfolgen aber nach Festlegung des Abtastschemas, da die MRF-Messung mit dem festgelegten Abtastschema durchgeführt wird. Das Dictionary muss spätestens vor der Auswertung der MRF-Messung ausgewählt werden. (Die Auswertung der MRF-Messung verwendet das Dictionary)

Die Auswahl eines Dictionary erfolgt bevorzugt basierend auf einem Vergleich der Ergebnisse der Vorabaufnahme mit den Intensitätsverläufen eines speziellen Prescan-Dictionary. Dabei können speziell Intensitätsverläufe verglichen werden. Es kann aber auch die vorgenannte Parameterreichweite der aufgenommenen Kontraste den Ausschlag für die Auswahl geben.

Die Festlegung des Abtastschemas kann basierend auf der Parameterreichweite der aufgenommenen Kontraste und/oder aus einem Intensitätsverlauf erfolgen.

Bei der Vorabaufnahme könnte es sich um eine niedrig aufgelöste MRF-Messung mit wenigen Repetitionen handeln, die mit einem eigenen Prescan-Dictionary verknüpft ist. Beispielsweise umfasst eine normale MRF-Aufnahme pro Schicht 1000 bis 3000 Repetitionen. Eine Vorabaufnahme könnte z.B. nur 200 Repetitionen umfassen).

Beispielsweise ist der Wertebereich der Kenngrößen also auch die Parameterreichweite, deutlich abhängig von der zu untersuchenden Körperregion bzw. des zu untersuchenden Gewebes, wie in folgender Tabelle veranschaulicht wird:

**Tabelle 1**

| Körperregion | T2 | T1 |
|---|---|---|
| Gehirn | 60-90 ms | 800-1400 ms |
| Lunge | < 1 ms | 800-1400 ms |
| Leber | 35 ms | 800 ms |
| Muskel | 30 ms | 1400 ms |

Es kann also bei vier Dictionarys, jeweils eines für Gehirn, Lunge, Leber und Muskel, ein Dictionary basierend auf Maxima und Minima der T1 und T2-Werte ausgewählt werden. Weiterhin können für jede Körperregion weitere (Sub-)Dictionarys gebildet werden, die z.B. auf für bestimmte Krankheiten typische T1- und T2-Parameterspektren optimiert sind. Das Abtastschema kann ebenso festgelegt werden. Die Auswahl bzw. die Festlegung kann automatisch und/oder manuell erfolgen.
- Durchführung einer MRF-Messung mit dem festgelegten Abtastschema und MRF-Auswertung basierend auf dem ausgewählten Dictionary. Wie oben bereits ausgeführt wurde, kann das Abtastschema unabhängig vom Dictionary festgelegt werden. Mit Auswahl eines Dictionary ist aber auch das Abtastschema vorgegeben, welches für die Aufnahmen der MRF-Messung angewandt werden muss, damit die Intensitätsverläufe der Pixel der aufgenommenen Bilder mit denen des ausgewählten Dictionary vergleichbar sind.

Es ist dabei sehr empfehlenswert die Aufnahmen der MRF-Messung mit dem Abtastschema aufzunehmen und nicht später in diesem Schema aneinanderzureihen (auch wenn dies durchaus eine Möglichkeit darstellt), da die Aufnahmen der Bilder oftmals nicht (im Grunde nie) unabhängig voneinander sind. In einem solchen Ablauf der Aufnahme entspricht die Bildnummer auch der zeitlichen Reihenfolge der Aufnahmen und lässt einen Rückschluss des Zeitpunkts im Akquisitionschema zu.

Das erfindungsgemäße Verfahren ist nicht nur für T2/T1-Parameteroptimierungen geeignet, sondern kann auch auf die Optimierung anderer Parameter wie Protonendichte, Diffusivität, Magnetisierungstransfer etc. verwendet werden.

Eine erfindungsgemäße Vorrichtung zur Steuerung eines Magnetresonanztomographie-Systems im Rahmen einer MRF-Messung umfasst die folgenden Komponenten:
- Eine Erstellungseinheit ausgelegt zur Erstellung einer Dictionary-Gruppe und/oder eine Datenschnittstelle zum Empfang einer bereitgestellten Dictionary-Gruppe, wobei die Dictionary-Gruppe mindestens zwei Dictionarys umfasst, wobei jedes Dictionary eine Vielzahl von unterschiedlichen Intensitätsverläufen von Bildpunkten einer Serie von MRT-Aufnahmen mit einem spezifischen Abtastschema enthält.
- Eine Vorabaufnahmeeinheit ausgelegt zur Anfertigung einer Vorabaufnahme von MRT-Messungen. Diese MRT-Messungen können einfach so gestaltet sein, dass Aussagen über die Parameterreichweite getroffen werden können, sie können aber auch eine Mehrzahl von MRT-Aufnahmen mit variierenden Aufnahmeparameterwerten umfassen.

- Eine Ermittlungseinheit ausgelegt zur Ermittlung und Festlegung eines Abtastschemas basierend auf der Vorabaufnahme.
- Eine Auswahleinheit ausgelegt zur Auswahl eines Dictionarys aus der Dictionary-Gruppe basierend auf der Vorabaufnahme.
- Eine Messeinheit ausgelegt zur Durchführung einer MRF-Messung mit dem festgelegten Abtastschema und MRF-Auswertung basierend auf dem ausgewählten Dictionary.

Eine erfindungsgemäße Steuereinrichtung zur Steuerung eines Magnetresonanztomographie-Systems ist für die Durchführung eines erfindungsgemäßen Verfahrens ausgelegt und/oder umfasst eine erfindungsgemäße Vorrichtung.

Ein erfindungsgemäßes Magnetresonanztomographie-System umfasst eine erfindungsgemäße Steuereinrichtung.

Ein erfindungsgemäßes Verfahren zur Herstellung eines (insbesondere erfindungsgemäßen) Magnetresonanztomographie-Systems umfasst die folgenden Schritte:
- Aufbau des Magnetresonanztomographie-Systems,
- Bereitstellen einer Anzahl von Referenz Phantomen in dem Magnetresonanztomographie-System,
- Anfertigung einer Vielzahl von MRT-Aufnahmen von der Anzahl von Referenz-Phantomen mit variierenden Aufnahmeparametern,
- Erstellung einer Mehrzahl von Dictionarys von pixelweisen Intensitätsverläufen der MRT-Aufnahmen, wobei für ein Dictionary jeweils dasselbe Abtastschema verwendet wird,
- Abspeichern der Mehrzahl von Dictionarys als Dictionary-Gruppe zusammen mit einer Information für jedes Dictionary, welches Abtastschema verwendet worden ist, in einem Datenspeicher des Magnetresonanztomographie-Systems, wobei zusammen mit einem Dictionary zusätzlich eine Information abgespeichert wird, für welchen Untersuchungsbereich das Dictionary vorgesehen ist.

Im Rahmen der Herstellung von Dictionarys können diese aus konkreten Messwerten (z.B. an Referenztargets) abgeleitet werden, bevorzugt ist aber auch, die Dictionarys zu simulieren bzw. basierend auf einer Simulation ein optimales Dictionary zu finden.

Ein Großteil der zuvor genannten Komponenten der Vorrichtung bzw. der Steuereinrichtung, können ganz oder teilweise in Form von Softwaremodulen in einem Prozessor einer entsprechenden Vorrichtung bzw. Steuereinrichtung realisiert werden. Eine weitgehend softwaremäßige Realisierung hat den Vorteil, dass auch schon bisher verwendete Vorrichtungen bzw. Steuereinrichtungen auf einfache Weise durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in ein Rechensystem bzw. eine Speichereinrichtung einer Steuereinrichtung eines Magnetresonanztomographiesystems ladbar ist, mit Programmabschnitten, um alle Schritte des erfindungsgemäßen Verfahrens auszuführen, wenn das Programm in dem Rechensystem bzw. der Steuereinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen

Zum Transport zum Rechensystem bzw. zur Steuereinrichtung und/oder zur Speicherung an oder in dem Rechensystem bzw. der Steuereinrichtung kann ein computerlesbares Medium, beispielsweise ein Memorystick, eine Festplatte oder ein sonstiger transportabler oder fest eingebauter Datenträger dienen, auf welchem die von einem Rechensystem bzw. einer Rechnereinheit der Steuereinrichtung einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

Bevorzugt ist die Vorabaufnahme eine niedrigaufgelöste Mapping-Sequenz, eine speziell optimierte MRF-Trajektorie mit geringer räumlicher und/oder zeitlicher Auflösung oder eine Relaxometrie-Messung, bevorzugt ohne Ortsauflösung. Eine solche Vorabaufnahme ist in wenigen Sekunden aufgenommen im Vergleich zu einigen Minuten für die MRF-Messung. Was die Vorabaufnahme betrifft, z.B. die räumliche Auflösung, ist die Vorabaufnahme so gestaltet, dass für denselben Aufnahmebereich die Messzeit mindestens um einen Faktor 5 kleiner ist als die der eigentlichen MRF-Messung.

Gemäß einem bevorzugten Verfahren basiert die Ermittlung des Abtastschemas auf der Parameterreichweite der bei der Vorabaufnahme aufgenommenen Kontraste. Dies wird bereits oben genauer beschrieben. Dazu wird die Parameterreichweite, z.B. das Minimum und das Maximum, der untersuchungsobjektspezifischen Parameter, z.B. aus den aufgenommenen Kontrasten, ermittelt. Wie in Tabelle 1 gezeigt, variieren die Parameterreichweiten bei der Aufnahme verschiedener Körperbereiche erheblich. Liegt für einen bestimmten Bereich von Parameterreichweiten ein bestimmtes Abtastschema vor, so wird dieses ermittelt und für die MRF-Messung festgelegt. Liegt keines vor, so wird bevorzugt dasjenige Abtastschema für die MRF-Messung festgelegt, bei dem die Parameterreichweiten am nächsten an den ermittelten liegen. Es kann aber alternativ oder zusätzlich eine Nachricht ausgegeben werden, dass kein passendes Abtastschema ermittelt werden konnte.

Gemäß einem bevorzugten Verfahren wird im Rahmen einer Aufnahme, insbesondere für die MRF-Messung, für eine Mehrzahl von MRT-Aufnahmen gemäß des festgelegten Abtastschemas einer oder mehrere der folgenden Aufnahmeparameterwerte variiert:
- Flipwinkel,
- Phase des Flipwinkels,
- Echozeit,
- Repetitionszeit,
- Echozug (z.B. Anzahl der Echos),
- Anzahl von aufgenommenen Bildern einer Bilderserie und
- Partial Fourier-Faktor/Trajektorie.

Die Vorabaufnahme ist bevorzugt so gestaltet, dass ein Überblick über die Parameterreichweite der Dictionarys in der Dictionary-Gruppe bezüglich der aufgenommenen Kontraste erlangt wird. Da die Dictionarys in der Regel mit unterschiedlichen Abtastschemata aufgenommen worden sind, die die Parameterreichweite der untersuchungsobjektspezifischen Parameter (z.B. T1 und T2) bei Messungen an den unterschiedlichen Körperregionen berücksichtigen, ist es von Vorteil, wenn die Vorabmessung den Gesamtbereich der Parameterreichweiten aller Dictionarys überspannt. Die Vorabmessung ist dabei bevorzugt so gestaltet, dass sie Messungen zumindest im Bereich der Grenzen aller Parameterreichweiten der einzelnen Dictionarys umfasst. Auf diese Weise kann vorteilhaft entschieden werden, welches Abtastschema (ggf. eines Dictionary) für die MRF-Messung verwendet werden soll.

Gemäß einem bevorzugten Verfahren erfolgt zur Festlegung des Abtastschemas und/oder zur Auswahl des Dictionary eine manuelle Auswahl durch einen Benutzer. Diese Auswahlmöglichkeit steht bevorzugt zusätzlich zu einer automatischen Festlegung des Abtastschemas und/oder zur Auswahl des Dictionary zur Verfügung. Beispielsweise kann über ein Computerterminal ein Überblick über die Vorabmessung zusammen mit einem Überblick über die Dictionarys der Dictionary-Gruppe und/oder ein Überblick über die zur Verfügung stehenden Abtastschemata ausgegeben werden und durch einen Benutzer ein gewünschtes Dictionary und/oder das gewünschte Abtastschema ausgewählt werden. Insbesondere bei erfahrenen Bedienern ist es vorteilhaft, wenn der Bediener eine manuelle Auswahl des Abtastschemas bzw. des Dictionary treffen kann.

Gemäß einem bevorzugten Verfahren ist eine Festlegung von zwei Arten von Abtastschemata für zumindest eine Art von MRF-Messung möglich. Damit ist gemeint, dass beide Abtastschemata für die betreffende MRF-Messung verwendet werden können und bevorzugt beide zum Vergleich der Messdaten mit demselben Dictionary verwendet werden können. Die eine Art von Abtastschema hat dabei eine höhere Auflösung als die andere Art von Abtastschema, und die andere Art von Abtastschema erlaubt eine schnellere Datenaufnahme als die erste Art von Abtastschema. Es gibt also zum Vergleich von Messdaten mit Einträgen eines Dictionary einmal eine "hochauflösende" Aufnahmemöglichkeit und einmal eine schnelle Aufnahmemöglichkeit.

Gleiches gilt für Dictionarys. Gemäß einem bevorzugten Verfahren ist eine Festlegung von zwei Arten von Dictionarys für zumindest eine Art von MRF-Messung möglich. Damit ist gemeint, dass beide Dictionarys für die betreffende MRF-Messung verwendet werden können. Die eine Art von Dictionary hat dabei eine höhere Auflösung als die andere Art von Dictionary, und die andere Art von Dictionary erlaubt eine schnellere Datenaufnahme als die erste Art von Dictionary. Es gibt also eine "hochaufgelöstes" Dictionary und einmal ein Dictionary für eine schnelle Aufnahme.

Beispielsweise liegen Abtastschemata und/oder Dictionarys vor, von denen ein Teil auf hohe T2/T1- Auflösung und ein anderer Teil auf eine schnelle Datenaufnahme bei reduzierter Genauigkeit optimiert ist. So kann insbesondere bei einem kleineren T1- bzw. T2-Wertebereich entweder die zur Aufnahme der Trajektorie benötigte Messzeit reduziert werden oder aber die Auflösung des abzutastenden Wertebereichs bei gleichbleibender Messzeit gesteigert werden. Weiterhin geht ein reduzierter T1- bzw. T2-Wertebereich mit einer reduzierten Rekonstruktionszeit beim Vergleich der Trajektorie mit den Dictionary-Einträgen einher.

Gemäß einem bevorzugten Verfahren werden für unterschiedliche Körperregionen und/oder Untersuchungen unterschiedliche Dictionarys und/oder unterschiedliche Abtastschemata verwendet. Diese Dictionarys bzw. Abtastschemata sind dabei bezüglich der betreffenden Körperregionen bzw. Untersuchungen optimiert, wobei bevorzugt Parameterreichweiten und/oder Kontraste und/oder bestimmte Variationen von Parameterwerten im Zuge eines Abtastschemas in Abhängigkeit von der Körperregion bzw. der Untersuchung angewandt werden.

Gemäß einem bevorzugten Verfahren wird nach Bereitstellung einer Dictionary-Gruppe vor der MRF-Messung ein zusätzliches Dictionary mit einem individuellen Abtastschema erstellt. Dies ist zwar zeitaufwändig, kann jedoch von Vorteil sein, wenn ein Patient in der zu untersuchenden Region vom Normalen abweichende Werte (z.B. anormale Parameterreichweiten) aufweist, z.B. aufgrund von Pathologien wie ein vaskularisierter bzw. nicht vaskularisierter Tumor oder Kalzifikationen. Mit solchen Pathologien kann es zu von den typischen Werten abweichenden Relaxationszeiten kommen.

Gemäß einem bevorzugten Verfahren werden zur Erstellung der Dictionary-Gruppe für unterschiedliche Untersuchungsregionen mittels Messungen spezifische Abtastschemata ermittelt. Bevorzugt findet alternativ oder zusätzlich für eine Untersuchungsregion eine für diese Untersuchungsregion spezifische Wahl von Parameterreichweiten statt.

Gemäß einem bevorzugten Verfahren wird zur Erstellung mehrerer Dictionarys einer Dictionary-Gruppe ein Referenz-Phantom verwendet, bevorzugt ein NIST-Phantom. Bei diesem Verfahren werden zusätzlich, bevorzugt im Vorfeld der MRF-Messung bzw. im Zuge des Aufbaus oder der Kalibrierung des MRT-Systems, die folgenden Schritte durchgeführt:
- Bereitstellen des Referenz-Phantoms in einem Magnetresonanztomographiesystem. Das Referenzphantom sollte dabei eine Simulation verschiedener Körperbereiche ermöglichen.
- Anfertigung einer Vielzahl von MRT-Aufnahmen von dem Referenz-Phantom mit variierenden Aufnahmeparametern. Diese variierenden Aufnahmeparameter sind bevorzugt variierte Flipwinkel, Echozeit, Partial Fourier-Faktor /Trajektorien, ein variierter TR-Zug oder Echozug.
- Automatisierte Bestimmung der Differenz zwischen den bekannten Referenzwerten des Referenz-Phantoms zu gemessenen Werten der MRT-Aufnahmen.
- Bestimmung des Mittelwerts sowie die Standardabweichung der gemessenen Werte. Dies dient zur Optimierung des Abtastschemas. Es wird dabei versucht, die vorangehend bestimmte Differenz zu minimieren. Die MRF-Messung liefert z.B. T1 und T2 Karten durch Ähnlichkeitsvergleich des Intensitätsverlaufs.
- Auswertung der Messungen für die zu optimierenden Körperregionen mit dazu passenden Bereichen des Referenz-Phantoms. Das Referenz-Phantom liefert eine hohe Bandbreite von T1 und T2 Werten. Wenn für eine Körperregion nur bestimmte Wertebereiche wichtig sind, dann wird die Minimierung der Differenz bevorzugt nur in den relevanten Teilen durchgeführt.

Gemäß einem weiteren bevorzugten Verfahren wird zur Erstellung mehrerer Dictionarys einer Dictionary-Gruppe eine Simulation von Intensitätsverläufen durchgeführt. Bevorzugt berücksichtigt diese Simulation auch die Artefakt- bzw. Bildqualitätsaspekte wie z.B. Rauschen oder Unterabtastung. Es ist bevorzugt, für verschiedene T1 und T2 Bereiche ein optimales Abtastschema zu finden, also ein Abtastschema, was am wenigsten Abweichungen zu den Referenzen zeigt. Eine Simulation basiert bevorzugt auf simulierten MRT-Abbildungen. Gemäß einer bevorzugten Alternative werden die Intensitätsverläufe analog zur Erstellung des Dictionarys durch eine Bloch-Simulation simuliert.

Die beschriebene Erweiterung mit angepassten Akquisitionen ist eine Möglichkeit MRF auf weite Regionen des menschlichen oder tierischen Körpers auszudehnen. Durch die Anpassung auf einen Wertebereich kann die Messung schneller durchgeführt werden. Z.B. die Anzahl der Echos oder der TR Zug wird je nach Region verringert. Und die Genauigkeit der Messung wird erhöht (T1 und T2 wird exakter bestimmt).

Die automatische Bestimmung des Abtastschemas ermöglicht ohne großen Aufwand, eine für eine vorbestimmte Körperregion optimierte Lösung zu finden.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
- Figur 1: einen Ablaufplan für einen möglichen Ablauf eines erfindungsgemäßen Verfahrens,
- Figur 2: einen beispielhaften Intensitätsverlauf,
- Figur 3: eine schematische Darstellung eines Magnetresonanztomographie-Systems gemäß einem Ausführungsbeispiel der Erfindung,
- Figur 4: ein bevorzugtes Verfahren zur Herstellung eines Magnetresonanztomographie-Systems,
- Figur 5: ein bevorzugtes Verfahren zur Erstellung einer Dictionary-Gruppe.

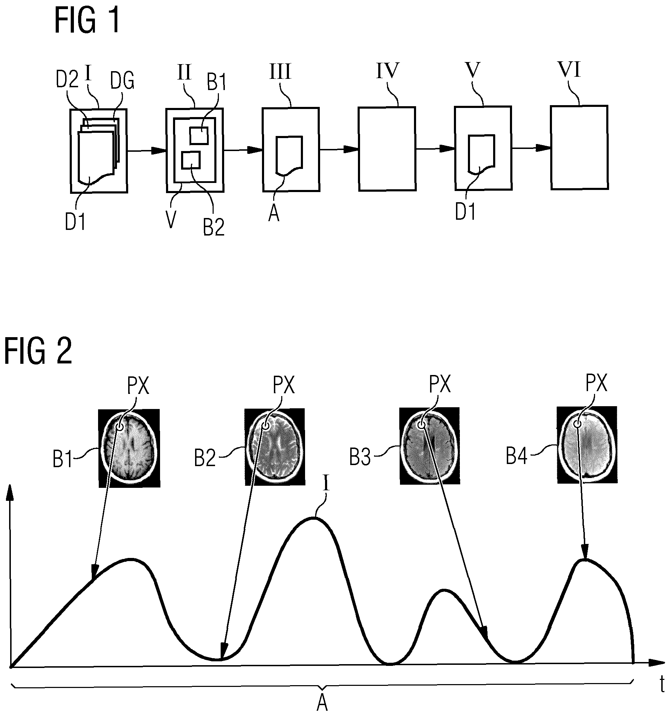

Figur 1 zeigt einen Ablaufplan für einen möglichen Ablauf eines erfindungsgemäßen Verfahrens zur Steuerung eines Magnetresonanztomographie-Systems im Rahmen einer MRF-Messung.

In Schritt I erfolgt eine Bereitstellung einer Dictionary-Gruppe DG umfassend mindestens zwei Dictionarys D1, D2, wobei jedes Dictionary D1, D2 eine Vielzahl von unterschiedlichen Intensitätsverläufen (s. Fig. 2) einer Serie von MRT-Aufnahmen B1, B2 mit einem spezifischen Abtastschema A enthält. Das Dictionary enthält Intensitätsverläufe die einen T1+ T2 Tupel und optional weiteren Parameter zugeordnet sind.

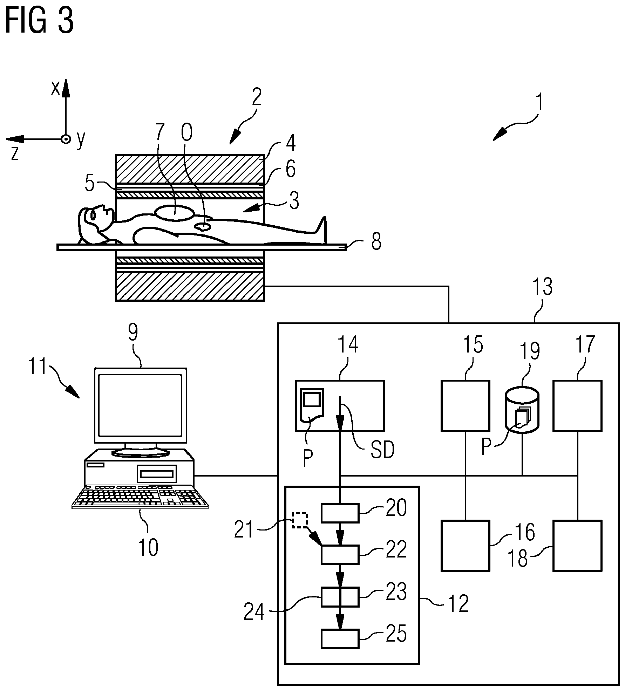

Im Rahmen dieses Schrittes kann auch eine Erstellung einer Dictionary-Gruppe DG erfolgen. Da dies jedoch sehr lange dauert, sollte dies zu einer Zeit durchgeführt werden, während der keinen weiteren Messungen durchgeführt werden, z.B. in der Nacht vor einer Messung oder sogar während des Aufstellens oder Zusammenbaus des betreffenden Magnetresonanztomographie-Systems 1 (s. Figur 3) oder alternativ durch Auslagern auf einen Rechencluster.

In Schritt II erfolgt eine Anfertigung einer Vorabaufnahme V. Diese MRT-Messungen können einfach so gestaltet sein, dass Aussagen über die Parameterreichweite getroffen werden können, sie können aber auch eine Mehrzahl von MRT-Aufnahmen B1, B2 mit variierenden Aufnahmeparameterwerten umfassen.

In Schritt III erfolgt eine Ermittlung und Festlegung eines Abtastschemas A basierend auf der Vorabaufnahme V.

In Schritt IV erfolgt eine Durchführung einer MRF-Messung mit dem festgelegten Abtastschema A.

In Schritt V erfolgt eine Auswahl eines Dictionary D1 aus der Dictionary Gruppe DG basierend auf der Vorabaufnahme V. In dem hier dargestellten Verfahren werden also die Festlegung des Abtastschemas A und die Auswahl des Dictionary D1 zu verschiedenen Zeiten vorgenommen. Es muss aber darauf geachtet werden, dass das Abtastschema A und das Dictionary D1 zusammenpassen, also die Einträge des Dictionary D1 auf dem Abtastschema A basieren.

In Schritt VI erfolgt eine MRF-Auswertung basierend auf dem ausgewählten Dictionary D1.

Figur 2 zeigt einen beispielhaften Intensitätsverlauf I über die Zeitachse t, der bei der Aufnahme von MRT-Aufnahmen B1, B2, B3, B4 mit einem bestimmten Abtastschema A entstehen kann. Ein solcher Intensitätsverlauf I wird bei einer MRF-Messung gemessen. Die Einträge der Dictionarys D1, D2 sind ebensolche Intensitätsverläufe I, die z.B. an einem Referenztarget gemessen worden sind und mit einem bestimmten Ergebnis, z.B. einer bestimmten Gewebeart datentechnisch verknüpft sind. An den MRT-Aufnahmen B1, B2, B3, B4 ist jeweils ein Pixel PX markiert (Kreis), der stets denselben Raumpunkt im Objekt markiert (hier ein Kopf). Da alle Bilder denselben Bereich zeigen, liegt der Pixel PX auch immer an derselben Bildkoordinate. Die unterschiedlichen Intensitäten der entsprechenden Pixel PX in den verschiedenen MRT-Aufnahmen B1, B2, B3, B4 ergeben den Intensitätsverlauf I.

In Figur 3 ist grob schematisch ein Magnetresonanztomographiesystem 1 dargestellt. Es umfasst zum einen den eigentlichen Magnetresonanzscanner 2 mit einem Untersuchungsraum 3 bzw. Patiententunnel, in den auf einer Liege 8 ein Patient oder Proband positioniert ist, in dessen Körper sich das eigentliche Untersuchungsobjekt O befindet, das auch als "Untersuchungsregion" O bezeichnet werden kann.

Der Magnetresonanzscanner 2 ist in üblicher Weise mit einem Grundfeldmagnetsystem 4, einem Gradientensystem 6 sowie einem HF-Sendeantennensystem 5 und einem HF-Empfangsantennensystem 7 ausgestattet. In dem dargestellten Ausführungsbeispiel handelt es sich bei dem HF-Sendeantennensystem 5 um eine im Magnetresonanzscanner 2 fest eingebaute Ganzkörperspule, wogegen das HF-Empfangsantennensystem 7 aus am Patienten bzw. Probanden anzuordnenden Lokalspulen besteht (in der Figur nur durch eine einzelne Lokalspule symbolisiert). Grundsätzlich können aber auch die Ganzkörperspule als HF-Empfangsantennensystem und die Lokalspulen als HF-Sendeantennensystem genutzt werden, sofern diese Spulen jeweils in unterschiedliche Betriebsweisen umschaltbar sind. Das Grundfeldmagnetsystem 4 ist hier in üblicher Weise so ausgebildet, dass es ein Grundmagnetfeld in Längsrichtung des Patienten erzeugt, d. h. entlang der in z-Richtung verlaufenden Längsachse des Magnetresonanzscanners 2. Das Gradientensystem 6 umfasst in üblicher Weise einzeln ansteuerbare Gradientenspulen, um unabhängig voneinander Gradienten in x-, y- oder z-Richtung schalten zu können.

Bei dem in Figur 3 dargestellten Magnetresonanztomographie-System handelt es sich um eine Ganzkörperanlage mit einem Patiententunnel, in den ein Patient komplett eingebracht werden kann. Grundsätzlich kann die Erfindung aber auch an anderen Magnetresonanztomographie-Systemen, z. B. mit seitlich offenem, C-förmigen Gehäuse, verwendet werden. Wesentlich ist nur, dass entsprechende Aufnahmen des Untersuchungsobjekts O angefertigt werden können.

Das Magnetresonanztomographie-System 1 weist weiterhin eine zentrale Steuereinrichtung 13 auf, die zur Steuerung des MR-Systems 1 verwendet wird. Diese zentrale Steuereinrichtung 13 umfasst eine Sequenzsteuereinheit 14. Mit dieser wird die Abfolge von Hochfrequenz-Pulsen (HF-Pulsen) und von Gradientenpulsen in Abhängigkeit von einer gewählten Pulssequenz PS oder einer Abfolge von mehreren Pulssequenzen zur Aufnahme mehrerer Schichten in einem interessierenden Volumenbereich des Untersuchungsobjekts innerhalb einer Messsitzung gesteuert. Eine solche Pulssequenz PS kann beispielsweise innerhalb eines Mess- oder Steuerprotokolls P vorgegeben und parametrisiert sein. Üblicherweise sind verschiedene Steuerprotokolle P für unterschiedliche Messungen bzw. Messsitzungen in einem Speicher 19 hinterlegt und können von einem Bediener ausgewählt (und bei Bedarf gegebenenfalls geändert) und dann zur Durchführung der Messung genutzt werden. Im vorliegenden Fall existieren Steuerprotokolle zur Aufnahme einer Vielzahl von MRT-Aufnahmen B1, B2, B3, B4 mit variierten Aufnahmeparametern.

Zur Ausgabe der einzelnen HF-Pulse einer Pulssequenz PS weist die zentrale Steuereinrichtung 13 eine Hochfrequenzsendeeinrichtung 15 auf, die die HF-Pulse erzeugt, verstärkt und über eine geeignete Schnittstelle (nicht im Detail gezeigt) in das HF-Sendeantennensystem 5 einspeist. Zur Steuerung der Gradientenspulen des Gradientensystems 6, um entsprechend der vorgegebenen Pulssequenz PS die Gradientenpulse passend zu schalten, weist die Steuereinrichtung 13 eine Gradientensystemschnittstelle 16 auf.

Die Steuereinrichtung 13 weist außerdem eine (ebenfalls in geeigneter Weise mit der Sequenzsteuereinheit 14 kommunizierende) Hochfrequenzempfangseinrichtung 17 auf, um innerhalb der durch die Pulssequenz PS vorgegebenen Auslesefenster koordiniert mittels des HF-Empfangsantennensystems 7 Magnetresonanz-Signale zu empfangen und so die Rohdaten zu akquirieren.

Eine Rekonstruktionseinheit 18 übernimmt hier die akquirierten Rohdaten und rekonstruiert daraus MRT-Aufnahmen B1, B2, B3, B4. Auch diese Rekonstruktion erfolgt in der Regel auf Basis von Parametern, die in dem jeweiligen Mess- oder Steuerprotokoll P vorgegeben sein können. Diese Bilddaten können dann beispielsweise in einem Speicher 19 hinterlegt werden.

Wie im Detail durch ein Einstrahlen von HF-Pulsen und das Schalten von Gradientenpulsen geeignete Rohdaten akquiriert und daraus MR-Bilder oder Parameter-Karten rekonstruiert werden können, ist dem Fachmann grundsätzlich bekannt und wird daher hier nicht näher erläutert.

Die Steuereinrichtung 13 des dargestellten Magnetresonanztomographie-Systems 1 umfasst eine bevorzugte Vorrichtung 12. Diese Vorrichtung 12 umfasst eine Datenschnittstelle 20 zum Empfang einer bereitgestellten Dictionary-Gruppe DG (s. Figur 1). Optional kann die Vorrichtung 12 auch eine Erstellungseinheit 21 ausgelegt zur Erstellung einer Dictionary-Gruppe DG umfassen. Des Weiteren umfasst die Vorrichtung 12 eine Vorabaufnahmeeinheit 22 ausgelegt zur Anfertigung einer Vorabaufnahme V von MRT-Messungen mit einer Mehrzahl von MRT-Aufnahmen B1, B2, B3, B4 (s. Figur 2) mit variierenden Aufnahmeparameterwerten, eine Ermittlungseinheit 23 ausgelegt zur Ermittlung und Festlegung eines Abtastschemas A basierend auf der Vorabaufnahme V und eine Auswahleinheit 24 ausgelegt zur Auswahl eines Dictionarys D1, D2 aus der Dictionary-Gruppe DG basierend auf der Vorabaufnahme V, und eine Messeinheit 25 ausgelegt zur Durchführung einer MRF-Messung mit dem festgelegten Abtastschema A und MRF-Auswertung basierend auf dem ausgewählten Dictionary D1, D2. Die Messeinheit 25 ist hier eine separate Einheit, welche die anderen Komponenten der Steuereinrichtung 13 entsprechend steuern kann. Sie kann jedoch auch mit den bereits vorhandenen Komponenten der Steuereinrichtung 13 realisiert werden.

Eine Bedienung der zentralen Steuereinrichtung 13 kann über ein Terminal 11 mit einer Eingabeeinheit 10 und einer Anzeigeeinheit 9 erfolgen, über das somit auch das gesamte Magnetresonanztomographie-System 1 durch eine Bedienperson bedient werden kann. Auf der Anzeigeeinheit 9 können auch Magnetresonanztomographie-Bilder angezeigt werden, und mittels der Eingabeeinheit 10, gegebenenfalls in Kombination mit der Anzeigeeinheit 9, können Messungen geplant und gestartet und insbesondere Steuerprotokolle P ausgewählt und gegebenenfalls modifiziert werden.

Das erfindungsgemäße Magnetresonanztomographie-System 1 und insbesondere die Steuereinrichtung 13 können darüber hinaus noch eine Vielzahl von weiteren, hier nicht im Einzelnen dargestellten, aber üblicherweise an derartigen Anlagen vorhandenen Komponenten aufweisen, wie beispielsweise eine Netzwerkschnittstelle, um das gesamte System mit einem Netzwerk zu verbinden und Rohdaten und/oder Bilddaten bzw. Parameter-karten, aber auch weitere Daten, wie beispielsweise patientenrelevante Daten oder Steuerprotokolle, austauschen zu können.

Wie durch ein Einstrahlen von HF-Pulsen und die Erzeugung von Gradientenfeldern geeignete Rohdaten akquiriert und daraus Magnetresonanztomographie-Bilder rekonstruiert werden können, ist dem Fachmann grundsätzlich bekannt und wird hier nicht näher erläutert. Ebenso sind verschiedenste Messsequenzen, wie z. B. EPI-Messsequenzen oder andere Messsequenzen zur Erzeugung von diffusionsgewichteten Bildern, dem Fachmann vom Grundsatz her bekannt.

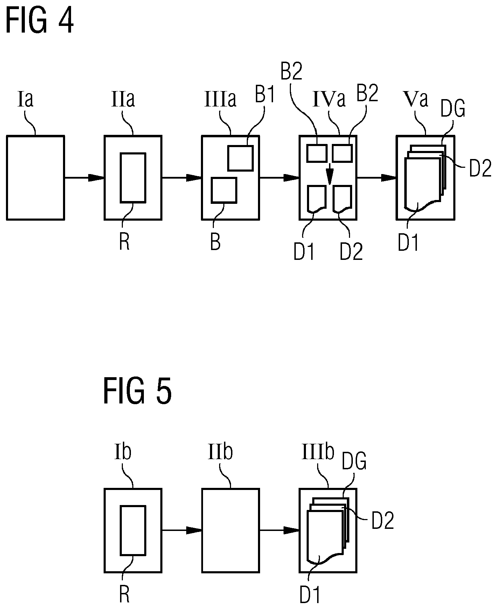

Figur 4 zeigt ein bevorzugtes Verfahren zur Herstellung eines Magnetresonanztomographie-Systems.

In Schritt Ia erfolgt ein Aufbau eines Magnetresonanztomographie-Systems 1, wie es z.B. in Figur 1 dargestellt ist. Dieses Magnetresonanztomographie-System umfasst eine Erstellungseinheit 21 ausgelegt zur Erstellung einer Dictionary-Gruppe DG wie vorangehend beschrieben.

In Schritt IIa erfolgt ein Bereitstellen einer Anzahl von Referenz-Phantomen R in dem Magnetresonanztomographie-System 1.

In Schritt IIIa erfolgt eine Anfertigung einer Vielzahl von MRT-Aufnahmen B1, B2, B3, B4 von der Anzahl von Referenz-Phantomen R mit variierenden Aufnahmeparametern.

In Schritt IVa erfolgt eine Erstellung einer Mehrzahl von Dictionarys D1, D2 von pixelweisen Intensitätsverläufen I der MRT-Aufnahmen B1, B2, B3, B4, wobei für ein Dictionary D1, D2 jeweils dasselbe Abtastschema A verwendet wird.

In Schritt Va erfolgt ein Abspeichern der Mehrzahl von Dictionarys D1, D2 als Dictionary-Gruppe DG zusammen mit einer Information für jedes Dictionary D1, D2, welches Abtastschema A verwendet worden ist, in einem Datenspeicher 19 des Magnetresonanztomographie-Systems 1.

Figur 5 zeigt ein bevorzugtes Verfahren zur Erstellung einer Dictionary-Gruppe

In Schritt Ib wird ein NIST-Phantom R als Referenz-Phantom R in das Magnetresonanztomographie-System 1 (s. Figur 3) gelegt und eine MRF-Messung wird z.B. über Nacht mit variierendem Flipwinkel, TE, TR-Zug bzw. Echozug und Partial Fourier-Faktor/Trajektorie ausgeführt.

In Schritt IIb werden Differenzen der bekannten Werte des NIST-Phantoms R zu den gemessenen Werten bestimmt. Die Werte des NIST-Phantoms R sind vorher exakt bekannt. Die Differenz kann im Nachhinein durch einfache Vergleiche automatisiert bestimmt werden. Die einzelnen Elemente (Kugeln) des NIST-Phantoms R liefern abseits des Rauschens konstante Werte. Ein gutes Maß zur statistischen Bearbeitung der Differenzen ist hier der Mittelwert sowie die Standardabweichung. Die T2 und T1 Werte des NIST Phantoms decken einen großen Bereich ab.

In Schritt IIIb werden für die Bewertung von einzelnen T2 und T1 Bereichen dann nur die Kugeln mit den für die jeweilige Region interessierenden T2 und T1 Werte ausgewertet und aus unterschiedlichen Konfigurationen der Auswertung Dictionarys D1, D2 erstellt.

Es wird abschließend noch einmal darauf hingewiesen, dass es sich bei den vorhergehend detailliert beschriebenen Verfahren sowie bei dem dargestellten Magnetresonanztomographiesystem 1 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Steuerung eines Magnetresonanztomographie-Systems (1) im Rahmen einer MRF-Messung umfassend die Schritte:
- Erstellung oder Bereitstellung einer Dictionary-Gruppe (DG) umfassend mindestens zwei Dictionarys (D1, D2), wobei jedes Dictionary (D1, D2) eine Vielzahl von unterschiedlichen Intensitätsverläufen (I) mit einem spezifischen Abtastschema (A) enthält,
- Anfertigen einer Vorabaufnahme (V) von MRT-Messungen,
- Ermittlung und Festlegung eines Abtastschemas (A) basierend auf der Vorabaufnahme (V),
- Auswahl eines Dictionary (D1, D2) aus der Dictionary Gruppe (DG) basierend auf der Vorabaufnahme (V),
- Durchführung einer MRF-Messung mit dem festgelegten Abtastschema (A) und MRF-Auswertung basierend auf dem ausgewählten Dictionary (D1, D2).

2. Verfahren nach Anspruch 1, wobei die Vorabaufnahme (V) eine Relaxometrie-Messung, eine niedrigaufgelöste Mapping-Sequenz oder eine speziell optimierte MRF-Trajektorie mit sehr geringer räumlicher Auflösung ist.

3. Verfahren nach einem der vorangehenden Ansprüche, wobei die Ermittlung des Abtastschemas (A) auf der Parameterreichweite der bei der Vorabaufnahme (V) aufgenommenen Kontraste basiert,
wobei die Vorabaufnahme (V) bevorzugt so gestaltet ist, dass ein Überblick über die Parameterreichweiten der Dictionarys (D1, D2) in der Dictionary-Gruppe (DG) bezüglich der aufgenommenen Kontraste erlangt wird und/oder
wobei für eine Mehrzahl von MRT-Aufnahmen (B1, B2, B3, B4) gemäß des Abtastschemas einer oder mehrere der folgenden Aufnahmeparameterwerte variiert wird: Flipwinkel, Phase des Flipwinkels, Echozeit, Repetitionszeit, Echozug, Anzahl von aufgenommenen Bildern einer Bilderserie und Partial Fourier/Trajektorie.

4. Verfahren nach einem der vorangehenden Ansprüche, wobei zur Festlegung des Abtastschemas (A) und/oder zur Auswahl des Dictionary (D1, D2) eine manuelle Auswahl durch einen Benutzer erfolgt, wobei diese Auswahlmöglichkeit bevorzugt zusätzlich zu einer automatischen Festlegung des Abtastschemas (A) und/oder zur Auswahl des Dictionary (D1, D2) zur Verfügung steht.

5. Verfahren nach einem der vorangehenden Ansprüche, wobei eine Festlegung von zwei Arten von Abtastschemata (A) und/oder von zwei Arten von Dictionarys (D1, D2) für zumindest eine Art von MRF-Messung möglich ist,
wobei die eine Art von Abtastschema (A) eine höhere Auflösung als die andere Art von Abtastschema (A) hat und die andere Art von Abtastschema (A) eine schnellere Datenaufnahme als die erste Art von Abtastschema (A) erlaubt und/oder wobei die eine Art von Dictionary (D1, D2) eine höhere Auflösung als die andere Art von Dictionary (D1, D2) hat und die andere Art von Dictionary (D1, D2) eine schnellere Datenaufnahme als die erste Art von Dictionary (D1, D2) erlaubt.

6. Verfahren nach einem der vorangehenden Ansprüche, wobei für unterschiedliche Körperregionen und/oder Untersuchungen unterschiedliche Dictionarys (D1, D2) und/oder unterschiedliche Abtastschemata (A) verwendet werden.

7. Verfahren nach einem der vorangehenden Ansprüche, wobei nach Bereitstellung einer Dictionary-Gruppe (DG) vor der MRF-Messung ein zusätzliches Dictionary (D1, D2) mit einem individuellen Abtastschema (A) erstellt wird.

8. Verfahren nach einem der vorangehenden Ansprüche, wobei zur Erstellung der Dictionary-Gruppe (DG) für unterschiedliche Untersuchungsregionen (O) mittels Messungen spezifische Abtastschemata (A) ermittelt werden und bevorzugt für eine Untersuchungsregion (O) eine für diese Untersuchungsregion spezifische Wahl von Parameterreichweiten stattfindet.

9. Verfahren nach einem der vorangehenden Ansprüche, wobei zur Erstellung mehrerer Dictionarys (D1, D2) einer Dictionary-Gruppe (DG) Intensitätsverläufe simuliert werden und/oder ein Referenz-Phantom (R), bevorzugt ein NIST-Phantom (R), verwendet wird und die folgenden Schritte durchgeführt werden:
- Bereitstellen des Referenz Phantoms (R) in einem Magnetresonanztomographie-System (1),
- Anfertigung einer Vielzahl von MRT-Aufnahmen (B1, B2, B3, B4) von dem Referenz-Phantom (R) mit variierenden Aufnahmeparametern, bevorzugt mit variierten Flipwinkeln, Partial Fourier-Faktor/Trajektorien, variierter Echozeit variiertem TR-Zug oder Echozug,
- automatisierte Bestimmung der Differenz zwischen den bekannten Referenzwerten des Referenz-Phantoms (R) zu gemessenen Werten der MRT-Aufnahmen (B1, B2, B3, B4),
- Bestimmung des Mittelwerts sowie die Standardabweichung der gemessenen Werte,
- Auswertung der Messungen für die zu optimierenden Körperregionen mit dazu passenden Bereichen des Referenz-Phantoms (R).

10. Vorrichtung zur Steuerung eines Magnetresonanztomographie-Systems (1) im Rahmen einer MRF-Messung umfassend:
- eine Erstellungseinheit (21), ausgelegt zur Erstellung einer Dictionary-Gruppe (DG) und/oder eine Datenschnittstelle (20) zum Empfang einer bereitgestellten Dictionary-Gruppe (DG), wobei die Dictionary-Gruppe (DG) mindestens zwei Dictionarys (D1, D2) umfasst, wobei jedes Dictionary (D1, D2) eine Vielzahl von unterschiedlichen Intensitätsverläufen (I) von Bildpunkten einer Serie von MRT-Aufnahmen (B1, B2, B3, B4) mit einem spezifischen Abtastschema (A) enthält,
- eine Vorabaufnahmeeinheit (22), ausgelegt zur Anfertigen einer Vorabaufnahme (V) von MRT-Messungen,
- eine Ermittlungseinheit (23), ausgelegt zur Ermittlung und Festlegung eines Abtastschemas (A) basierend auf der Vorabaufnahme (V),
- eine Auswahleinheit (24), ausgelegt zur Auswahl eines Dictionarys (D1, D2) aus der Dictionary-Gruppe (DG) basierend auf der Vorabaufnahme (V),
- eine Messeinheit (25), ausgelegt zur Durchführung einer MRF-Messung mit dem festgelegten Abtastschema (A) und MRF-Auswertung basierend auf dem ausgewählten Dictionary (D1, D2).

11. Steuereinrichtung (13) zur Steuerung eines Magnetresonanztomographie-Systems (1), welche zur Durchführung eines Verfahrens nach einem der Ansprüche 1 bis 9 ausgestaltet ist und/oder welche eine Vorrichtung (12) gemäß Anspruch 10 umfasst.

12. Magnetresonanztomographie-System (1) umfassend eine Steuereinrichtung (13) nach Anspruch 11.

13. Verfahren zur Herstellung eines Magnetresonanztomographie-Systems (1), insbesondere nach Anspruch 12, umfassend die Schritte:
- Aufbau des Magnetresonanztomographie-Systems (1),
- Bereitstellen einer Anzahl von Referenz-Phantomen (R) in dem Magnetresonanztomographie-System (1),
- Anfertigung einer Vielzahl von MRT-Aufnahmen (B1, B2, B3, B4) von der Anzahl von Referenz-Phantomen (R) mit variierenden Aufnahmeparametern,
- Erstellung einer Mehrzahl von Dictionarys (D1, D2) von pixelweisen Intensitätsverläufen (I) der MRT-Aufnahmen (B1, B2, B3, B4), wobei für ein Dictionary (D1, D2) jeweils dasselbe Abtastschema (A) verwendet wird,
- Abspeichern der Mehrzahl von Dictionarys (D1, D2) als Dictionary-Gruppe (DG) zusammen mit einer Information für jedes Dictionary (D1, D2), welches Abtastschema (A) verwendet worden ist, in einem Datenspeicher (19) des Magnetresonanztomographie-Systems (1), wobei zusammen mit einem Dictionary zusätzlich eine Information abgespeichert wird, für welchen Untersuchungsbereich das Dictionary vorgesehen ist.

14. Computerprogrammprodukt mit einem Computerprogramm, welches direkt in eine Speichereinrichtung einer Steuereinrichtung eines Magnetresonanztomographie-Systems (1) ladbar ist, mit Programmabschnitten, um alle Schritte des Verfahrens nach einem der Ansprüche 1 bis 9 auszuführen, wenn das Computerprogramm in der Steuereinrichtung des Magnetresonanztomographie-Systems (1) ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 9 auszuführen, wenn die Programmabschnitte von der Rechnereinheit ausgeführt werden.
